# EUROPEAN PATENT APPLICATION

(11) **EP 1 992 316 A2**
(43) Date of publication of application: **19.11.2008**
(21) Application number: 08251714.5
(22) Date of filing: 15.05.2008
(51) Int. Cl.: A61F 5/00

(54) **Gastric band with position sensing**

(30) Priority: 16.05.2007 US 749387
(71) Applicant: Biosense Webster, Inc., Diamond Bar, CA 91765 (US)
(72) Inventor: Ephrath, Yaron, Karkur 35170 (IL); Govari, Assaf, Haifa 34400 (IL); Altmann, Andres, Haifa 34614 (IL); Schwartz, Yitzhack, Haifa 34606 (IL)
(74) Representative: Small, Gary James

(57) **Abstract**

The problem of accessing an injection port transcutaneously is resolved using wireless position transducers (34,36) in an inflation port assembly (26) and in an injection syringe (28). The measurements provided by the transducers indicate to the practitioner the position and orientation of syringe relative to the injection port. A console (46) provides a visual indication of the relative position and orientation so as to guide the practitioner to insert the syringe at the proper site and in the proper direction and to penetrate the port cleanly and correctly.

## Description

### BACKGROUND OF THE INVENTION

### Field of the Invention

This invention relates to determining the positions of objects inside a living body. More particularly, this invention relates to determining the position and alignment of an injector relative to an injection port located inside a living body.

### Description of the Related Art

Gastric bands are used to restrict food intake in cases of morbid obesity. An inflatable gastric band is inserted surgically so as to encircle a portion of a patient's stomach. The band forms a small proximal pouch with a constricted stoma that allows food to slowly pass therethrough. The band may be inflated or deflated by a medical practitioner in order to adjust the size of the stoma and thus control the patient's food intake.

In typical gastric band systems, the band is connected by a tube to an inflation port near the body surface. To inflate or deflate the band, the practitioner inserts a syringe into the port and either injects or withdraws fluid through the port. Finding the port is often difficult, particularly in very obese patients, and may require a substantial amount of trial and error. This is inconvenient to the patient, and often produces substantial discomfort.

U.S. Patent No. 6,450,946, issued to Forsell, proposes to restrict food intake using a restriction device implanted in a patient and engaging the stomach or the esophagus to form an upper pouch of the stomach and a restricted stoma or passage in the stomach or esophagus. An energy transmission device for wireless transmission of energy of a first form from outside the patient's body is provided. An implanted energy transfer device transfers the energy of the first form transmitted by the energy transmission device into energy of a second form, different from the first form. The energy of the second form is used to control the operation of the restriction device to vary the size of the restricted passage.

U.S. Patent No. 6,305,381, issued to Weijand, et al., describes a system and method for locating an implantable medical device. The system consists of a flat "pancake" antenna coil positioned concentric with the implantable medical device target, e.g., a drug reservoir septum. The system further features an antenna array, which is separate from the implantable device and external to the patient. The antenna array features three or more separate antennas, which are used to sense the energy emitted from the implanted antenna coil. The system further features a processor to process the energy ducted by the antenna array. The system senses the proximity to the implant coil and, thus, the implant device by determining when an equal amount of energy is present in each of the antennas of the antenna array and if each such ducted energy is greater than a predetermined minimum. When such a condition is met, the antenna array is aligned with the implant coil.

U.S. Patent Nos. 5,391,199 and 5,443,489, issued to Ben-Haim, whose disclosures are incorporated herein by reference, describe systems wherein the coordinates of an intrabody probe are determined using one or more field sensors, such as a Hall effect device, coils, or other antennae carried on the probe. Such systems are used for generating three-dimensional location information regarding a medical probe or catheter. Preferably, a sensor coil is placed in the catheter and generates signals in response to externally applied magnetic fields. The magnetic fields are generated by three radiator coils, fixed to an external reference frame in known, mutually spaced locations. The amplitudes of the signals generated in response to each of the radiator coil fields are detected and used to compute the location of the sensor coil. Each radiator coil is preferably driven by driver circuitry to generate a field at a known frequency, distinct from that of other radiator coils, so that the signals generated by the sensor coil may be separated by frequency into components corresponding to the different radiator coils.

U.S. Patent No. 6,198,963, issued to Ben-Haim et al., whose disclosure is incorporated herein by reference, describes simplified apparatus for confirmation of intrabody tube location that can be operated by nonprofessionals. The initial location of the object is determined as a reference point, and subsequent measurements are made to determine whether the object has remained in its initial position. Measurements are based upon one or more signals transmitted to and/or from a sensor fixed to the body of the object whose location is being determined. The signal could be ultrasound waves, ultraviolet waves, radio frequency (RF) waves, or static or rotating electromagnetic fields.

### SUMMARY OF THE INVENTION

According to disclosed embodiments of the invention, the problem of transcutaneously accessing the injection port of an inflatable restriction device is solved by using wireless position transponders in the inflation port assembly and in an injection device that is used to inflate and deflate the port. The signals provided by the transponders indicate to the practitioner the position and orientation of the injection device relative to the injection port. In some embodiments, a console provides a visual indication of the relative positions and alignment of the injection device and the port. The visual indication guides the practitioner in maneuvering the injection device so that it penetrates the port cleanly and correctly.

An embodiment of the invention provides a method for adjusting an inflatable gastric restriction device within a body of a living subject, which is carried out by disposing a wireless transponder on the gastric restriction device. The wireless transponder generates a location signal relative to a receiver, which has a known relation to an injection device that is adapted to a port of the gastric restriction device. The method is further carried out by irradiating the wireless transponder with a driving field, the wireless transponder being powered at least in part by the driving field. The method is further carried out by wirelessly transmitting an output signal by the wireless transponder responsively to the driving field, receiving and processing the output signal to determine respective locations and orientations of the injection device and the port, and responsively to the respective locations and orientations, navigating the injection device within the body to introduce the injection device into the port, and changing a fluid content of the gastric restriction device using the injection device.

An aspect of the method includes disposing on the injection device a second wireless transponder that generates a second output signal, and generating a plurality of electromagnetic fields at respective frequencies in a vicinity of the wireless transponder and in a vicinity of the second wireless transponder, wherein the output signal and the second output signal include information indicative of respective strengths of the electromagnetic fields at the wireless transponder and the second wireless transponder.

One aspect of the method includes storing first electrical energy and second electrical energy derived from the driving field in the wireless transponder and the second wireless transponder, respectively, and transmitting the output signal and the second output signal using the first electrical energy and the second electrical energy, respectively.

According to one aspect of the method, the wireless transponder and the second wireless transponder are powered exclusively by the driving field.

An additional aspect of the method includes transmitting telemetry signals from the gastric restriction device, the telemetry signals containing information of a state of the gastric restriction device.

An embodiment of the invention provides a location system for adjusting an inflatable gastric restriction device within a living subject, The system includes an injection device that is receivable by a port of the gastric restriction device. The injection device has a second wireless transponder. The first wireless transponder and the second wireless transponder each comprise a position sensor, a transmitter for irradiating the first wireless transponder and the second wireless transponder with a driving field. The first wireless transponder and the second wireless transponder are each powered at least in part by the driving field to energize the position sensor thereof. The first wireless transponder and the second wireless transponder are operative responsively to the driving field for wirelessly transmitting a first output signal and a second output signal, respectively. The system further includes electrical circuitry for receiving and processing the first output signal and the second output signal to determine respective locations and orientations of the port and the injection device, and a console that is operative for displaying visual indications of the respective locations and orientations.

### BRIEF DESCRIPTION OF THE DRAWINGS

For a better understanding of the present invention, reference is made to the detailed description of the invention, by way of example, which is to be read in conjunction with the following drawings, wherein like elements are given like reference numerals, and wherein:

Fig. 1 schematically illustrates a system for sensing a position and orientation of an injection or aspiration device relative to a port in accordance with a disclosed embodiment of the invention;

Fig. 2 schematically illustrates details of a wireless position transponder for use in the system shown in Fig. 1, in accordance with a disclosed embodiment of the invention;

Fig. 3 schematically shows details of driving and processing circuits in a processor in the system shown in Fig. 1, in accordance with a disclosed embodiment of the invention;

Fig. 4 is a block diagram showing details of an embodiment of the front end of a receiver in the circuitry shown in Fig. 3, which is adapted to receive signals from a plurality of transponders concurrently, in accordance with a disclosed embodiment of the invention;

Fig. 5 is a schematic diagram of a system for sensing a position and orientation of an injection or aspiration device relative to an injection port that is located within a body of a living subject, in accordance with an alternate embodiment of the invention;

Fig. 6 schematically illustrates a system for sensing a position and orientation of an injection or aspiration device relative to a port located in a living subject in accordance with an alternate embodiment of the invention;

Fig. 7 schematically illustrates details of a wireless position transponder, in accordance with an alternate embodiment of the invention;

Fig. 8 schematically shows details of a wireless transponder in accordance with an alternate embodiment of the invention;

Fig. 9 is a block diagram of driving and processing circuitry, which are cooperative with the transponder shown in Fig. 8, in accordance with a disclosed embodiment of the present invention;

Fig. 10 is a flow chart of a method for transmitting a digital signal, using the transponder and circuitry shown in Fig. 8 and Fig. 9, in accordance with a disclosed embodiment of the invention;

Fig. 11 is a block diagram of driving and processing circuitry, which are cooperative with the transponder shown in Fig. 8, in accordance with an alternate embodiment of the present invention; and

Fig. 12 is a block diagram of a wireless position transponder in accordance with an alternate embodiment of the invention.

### DETAILED DESCRIPTION OF THE INVENTION

In the following description, numerous specific details are set forth in order to provide a thorough understanding of the present invention. It will be apparent to one skilled in the art, however, that the present invention may be practiced without these specific details. In other instances, well-known circuits, and control logic have not been shown in detail in order not to obscure the present invention unnecessarily.

### Embodiment 1

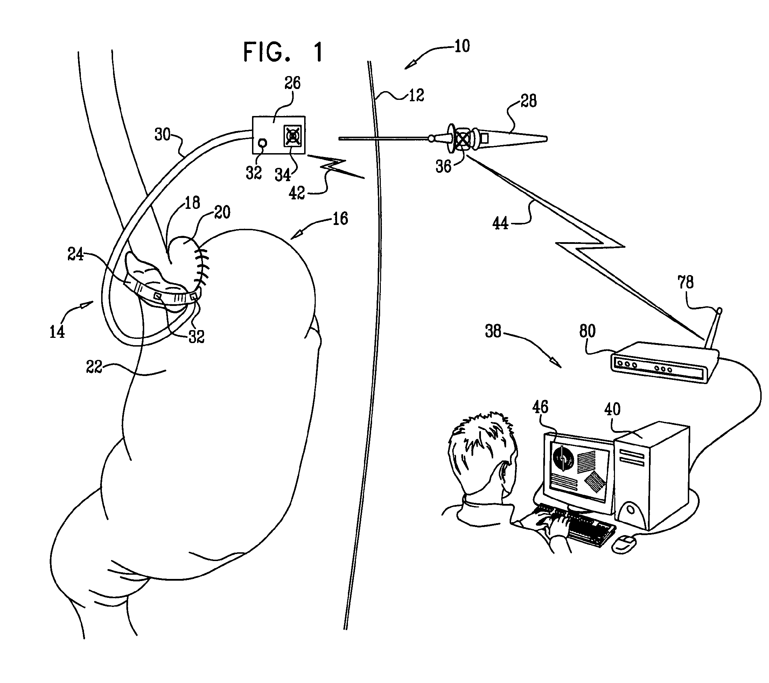

Turning now to the drawings, reference is initially made to Fig. 1, which schematically illustrates a system 10 for sensing a position and orientation of an injection or aspiration device relative to a port of injection and aspiration of fluid that is located within a body of a living subject, in accordance with a disclosed embodiment of the invention. A body surface 12 is represented by a vertical line. A generic inflatable gastric restriction device 14 is emplaced on a stomach 16 near its esophagogastric junction 18. The restriction device 14 constricts the gastric lumen, segmenting the stomach 16 into proximal portion 20 and a distal portion 22. The restriction device 14 creates a relatively narrow stoma or passage, which retards the movement of food from the proximal portion 20 to the distal portion 22.

In the restriction device 14, a band 24 engages and at least partially wraps around the stomach 16. An inflation port 26, usually disposed near the body surface 12, is adapted to receive an injection device, which is typically a syringe 28. Typically, a tube 30 connects the inflation port 26 with the band 24. To inflate or deflate the band 24, and thereby respectively enlarge or constrict the passage, the practitioner inserts the syringe 28 into the inflation port 26, and injects or withdraws fluid, as the case may be. Finding the inflation port 26 is often difficult, particularly in very obese patients, and may require a substantial amount of trial and error. The band 24 and the inflation port 26 may include sensors 32 that measure such parameters as intraluminal pressure.

In order to position the syringe 28 in alignment with the inflation port 26, at least one transmitter is implanted on the restriction device 14, at the inflation port 26 or at least in a known relationship to the inflation port 26. A second transmitter may additionally be emplaced at the syringe 28, shown in Fig. 1 as wireless position transponders 34, 36, which in this example are affixed to the inflation port 26 and the syringe 28, respectively. The disposition of the transponders is not critical. They can be externally or internally located, so long as offsets between the transponders and points of interest on the inflation port 26 and the syringe 28 are known. Measurements derived from signals provided by the transponders indicate to the practitioner the position and orientation of the syringe 28 relative to the inflation port 26. Signals originating from the transponders 34, 36 are transmitted to a field receiving unit 38, which processes the transponder signals in order to determine the locations of the transponders 34, 36, and hence the locations of the inflation port 26 and the syringe 28. Typically, in the receiving unit 38, a processor 40 receives wireless signals 42, 44 from the transponders 34, 36, and after suitable signal processing, a console 46 displays a visual indication of the relative position and orientation of the syringe 28 and the inflation port 26. The display guides the practitioner to navigate the syringe 28 so as to penetrate the body surface 12 and then reach the inflation port 26 correctly.

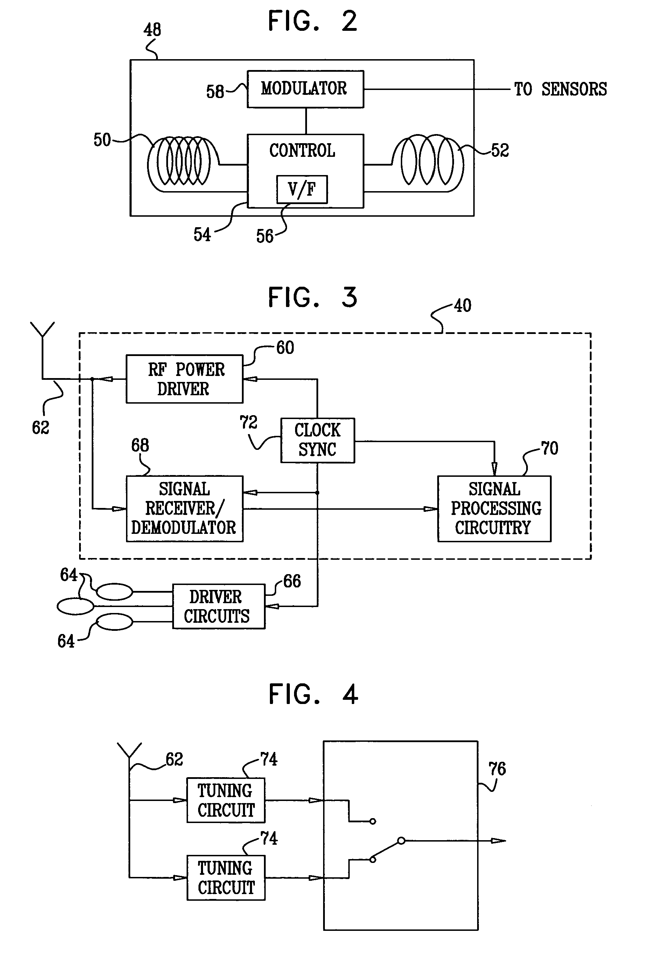

Reference is now made to Fig. 2, which schematically illustrates details of a wireless position transponder 48, which can be used as the transponders 34, 36 (Fig. 1), in accordance with a disclosed embodiment of the invention. The transponder 48 comprises a power coil 50 and a sensing coil 52, coupled to control circuitry, typically embodied as a control chip 54. The control chip 54 comprises a voltage-to-frequency converter 56, which generates a RF signal whose frequency is proportional to the voltage produced by the current through the sensing coil 52 flowing across a load, which can be measured as a voltage drop across the sensing coil 52. Additional modulation can be imposed on the RF signals transmitted by the transponder 48, using a modulator 58. This allows incorporation of information taken from the sensors 32 (Fig. 1) into the transmitted signals. Any suitable modulation scheme may be employed by the modulator 58.

The power coil 50 is preferably optimized to receive and transmit high-frequency signals, in the range above 1 MHz. The sensing coil 52, on the other hand, is preferably designed for operation in the range of 1-3 kHz. As will be explained below, the sensing coil 52 is operationally disposed within an electromagnetic field having a frequency in the range of 1-3 kHz. Alternatively, other frequency ranges may be used, as dictated by application requirements. The entire transponder 48 is typically 2-5 mm in length and 2-3 mm in outer diameter, enabling it to be housed conveniently in the syringe 28 and the inflation port 26 (Fig. 1).

Reference is now made to Fig. 3, which schematically shows details of driving and processing circuits in the processor 40 of the receiving unit 38 (Fig. 1), in accordance with a disclosed embodiment of the invention. The processor 40 comprises a RF power driver 60, which drives an antenna 62 to emit a power signal, preferably in the 2-40 MHz range. Values in the Industrial, Scientific and Medical (ISM) bands of 13, 27, and 40 MHz have all been found to be suitable. A plurality of field generator coils 64, driven by driver circuitry 66, produce electromagnetic fields at different frequencies that energize the transponder 48 (Fig. 2), as explained below.

Referring again to Fig. 2, the power signal produced by the antenna 62 (Fig. 3) causes a current to flow in the power coil 50, which is rectified by the control chip 54 and used to power its internal circuits. The control chip 54 in the transponder 48 (Fig. 2) uses the RF signal received by the power coil 50 not only as its sole power source, but also as a frequency reference.

Meanwhile, electromagnetic fields produced by the field generator coils 64 (Fig. 3) cause a current to flow in the sensing coil 52. This current has frequency components at the same frequencies as the driving currents flowing through the field generator coils 64. The frequency components are proportional in amplitude to the strengths of the components of the respective magnetic fields produced by the field generator coils 64 in a direction parallel to the axis of the sensing coil 52. Thus, the amplitudes of the currents indicate the position and orientation of the sensing coil 52 relative to the field generator coils 64.

The control chip 54 measures the currents flowing in the sensing coil 52 at the different field frequencies. It encodes this measurement in a high-frequency signal, which it then transmits back via the power coil 50 to the antenna 62 (Fig. 3). Preferably, the RF signal produced by the control chip 54 has a carrier frequency in the range 50 MHz-2.5 GHz. ISM frequencies of 433,915 MHz and 2.5 GHz have been found to be suitable. The RF signal produced in this manner is modulated with three different frequency modulation (FM) components that vary over time at the respective frequencies of the fields generated by the field generator coils 64. The magnitude of the modulation is proportional to the current components at the three frequencies. An advantage of using frequency modulation, rather than amplitude modulation, to convey the sensor coil amplitude measurements from the transponder 48 to the antenna 62 is that the information in the signal (i.e., the frequency) is unaffected by the variable attenuation of the body tissues through which the signal must pass.

Referring again to Fig. 3, The signal transmitted by the power coil 50 (Fig. 2) is picked up by the antenna 62 and input to a receiver 68. The receiver 68 demodulates the signal to generate a suitable input for signal processing circuitry 70. Typically, the receiver 68 amplifies, filters and digitizes the signals from the transponder 48 (Fig. 2). The digitized signals are received and used by the signal processing circuitry 70 to compute the position and orientation of the transponder 48. Using pre-established offsets, the position and orientation of a structure connected to the transponder 48 can then be derived. The signal processing circuitry 70 may be realized as dedicated circuitry, or as a general-purpose computer, which is programmed and equipped with appropriate input circuitry for processing the signals from the receiver 68.

The processor 40 includes a clock synchronization circuit 72, which is used to synchronize the driver circuitry 66 and the power driver 60. Using the frequency reference provided by the power driver 60, both the control chip 54 in the transponder 48 (Fig. 2) and the receiver 68 are able to apply phase-sensitive processing as known in the art to the current signals generated by the sensing coil 52 (Fig. 2), in order to detect the current of the sensing coil 52 in phase with the driving fields generated by the field generator coils 64. In the case of the receiver 68, input is also taken from the clock synchronization circuit 72. Such phase-sensitive detection methods enable the transponder 48 to achieve an enhanced signal/noise ratio, despite the low amplitude of the current signals in the sensing coil 52.

A point of possible ambiguity in determining the orientation coordinates of the transponder 48 (Fig. 2) is that the magnitude of the currents flowing in the sensing coil 52 is invariant under reversal of the direction of the axis of the coil. In other words, flipping the transponder 48 by 180 degrees through a plane perpendicular to the axis of the sensing coil 52 has no effect on the current amplitude. Under some circumstances, this symmetrical response could cause an error of 180 degrees in determining the position and orientation coordinates of the transponder 48. This ambiguity is usually not relevant in practice, as the orientation is known from the operating environment.

While the magnitude of the current in the sensing coil 52 is unaffected by flipping the coil axis, the 180 degree reversal reverses the phase of the current relative to the phase of the electromagnetic fields generated by the field generator coils 64. The clock synchronization circuit 72 can be used to detect this phase reversal and thus overcome the ambiguity of orientation when 180 degree rotation occurs. Synchronizing the modulation of the RF signal returned by the control chip 54 (Fig. 2) to the receiver 68 with the driving currents of the field generator coils 64 enables the receiver 68 to determine the phase of the currents in the sensing coil 52 relative to the driving currents. By checking whether the sensor currents are in phase with the driving currents, or are 180 degrees out of phase, the signal processing circuitry 70 is able to decide in which direction the transponder 48 is pointing.

Reference is now made to Fig. 4, which is a block diagram showing details of an embodiment of the front end of the receiver 68 (Fig. 3), which is adapted to receive signals from both of the transponders 34, 36 (Fig. 1) concurrently, in accordance with a disclosed embodiment of the invention. Embodiments of the transponders 34, 36 may or may not transmit at different frequencies, or otherwise use different signatures. In any case, it is necessary for the receiver 68 (and the signal processing circuitry 70) to differentiate among the transponders. In the embodiment of Fig. 4, it is assumed that the frequencies emitted by the transponders 34, 36 are different. The antenna 62 is coupled to a plurality of tuning circuits 74, each tuned to a respective frequency emitted by one of the transponders 34, 36. A switch 76 time multiplexes the outputs of the tuning circuits 74, and directs them to further signal processing circuitry, as is known in the receiver art. Other multiplexing techniques known in the art may also be employed to allow a single receiver to process signals from a plurality of transponders.

Alternatively, it is possible to switch the signals of the transponders 34, 36 using many other switching circuits known in the art. Alternatively, components of the receiver 68 and the signal processing circuitry 70 could be duplicated and dedicated to transponders 34, 36, respectively. However, this alternative would generally be more expensive and hence, less satisfactory.

Further details of the transponder 48 (Fig. 2) and the processor 40 (Fig. 3) are described in PCT Publication WO 96/05768, the above-noted U.S. Patent No. 6,690,963, and in U.S. Patent Application Publication Nos. 2003/0120150 and 2005/0099290, the disclosures of which are herein incorporated by reference.

### Operation

Referring again to Fig. 1, to operate the system 10, a subject is placed in a magnetic field generated by the field generator coils 64 (Fig. 3). For example, the field generator coils 64 may be disposed in a pad disposed beneath the subject (not shown). A reference electromagnetic sensor (not shown) is preferably fixed relative to the patient, for example, taped to the patient's back, and the syringe 28 is advanced into the patient toward the inflation port 26. The processor 40 constantly updates the relative positions and orientations of the inflation port 26 and the syringe 28 and displays a visual indication on the console 46. Thus an operator is able at all times during the procedure to determine the precise location of the tip of the syringe 28 relative to the inflation port 26. When the inflation port 26 is suitably engaged by the syringe 28, fluid is injected or aspirated from the band 24 as required. Subsequently, the syringe 28 is withdrawn to terminate the operation.

### Embodiment 2

Referring again to Fig. 1, both of the transponders 34, 36 may be configured as transmitters, and their positions may be determined relative to a separate receiving location pad on the patient's body or fixed outside the body.

Alternatively, various position and orientation configurations may be used in the system 10. For example, one of the transponders 34, 36 may be configured as a magnetic field transmitter, while the other is configured as a receiver.

### Embodiment 3

Continuing to refer to Fig. 1, various sensors may be associated with the gastric band, such as a pressure sensor or temperature sensor. The magnetic field transducer associated with the inflation port 26 may then also be used as a data transmitter for purposes of telemetry, in order to transmit measurement values relating to the state of the gastric band to the console, e.g., the fluid pressure in the band 24. The telemetry signals may be received by a suitable receiver in the syringe 28 (not shown) or at a telemetry antenna 78 of a receiver unit 80, which can be a separate unit as shown in Fig. 1, or can be integrated in the processor 40.

### Embodiment 4

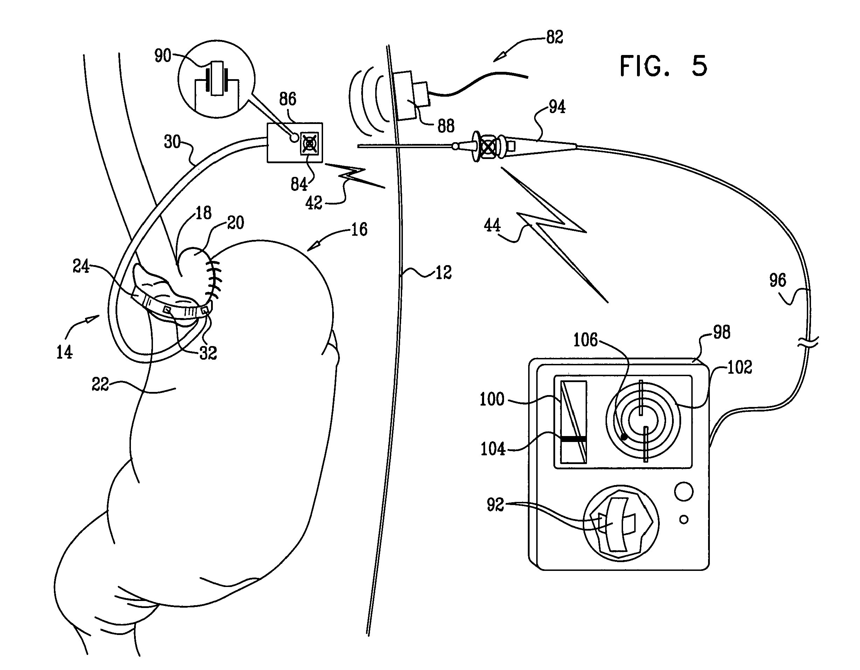

Other types of position sensing may be used, such as ultrasonic position sensing. Reference is now made to Fig. 5, which is a schematic diagram of a system 82 for sensing a position and orientation of an injection or aspiration device relative to an injection port that is located within a body of a living subject, in accordance with an alternate embodiment of the invention.

In this embodiment, a wireless transponder 84, attached to an injection port 86 located within the body of a patient, receives its operating power not from an electromagnetic field, but from acoustic energy generated by an ultrasound transmitter 88. A device of this sort is shown, for example, in U.S. Patent Application Publication No. 2003/0018246, the disclosure of which is herein incorporated by reference. The acoustic energy generated by the ultrasound transmitter 88 excites a miniature transducer, such as a piezoelectric crystal 90, in the wireless transponder 84, to generate electrical energy that powers the transponder. The electrical energy causes a current to flow in one or more coils in the wireless transponder 84, such as the power coil 50 (Fig. 2) described above. The currents in the coils in the wireless transponder 84 generate electromagnetic fields outside the patient's body, which are in this case received by field receivers 92. The amplitudes of the currents flowing in coils at the frequency of the applied acoustic energy are measured to determine the position of the wireless transponder 84 in relationship with an injection device or syringe 94, which contains a transponder as described above, which may be wireless or powered by a cable 96.

A display 98 preferably comprises a distance guide 100 and an orientation target 102. A mark 104 on the distance guide 100 indicates how far the tip of the syringe 94 is from the location of the port 86. A cursor 106 on the orientation target 102 indicates the orientation of tool 76 relative to the axis required to reach the port 86. When the cursor 106 is centered on the orientation target 102, it means that the syringe 94 is pointing directly toward the port 86. The console 46 (Fig. 1) preferably works on a similar principle.

### Embodiment 5

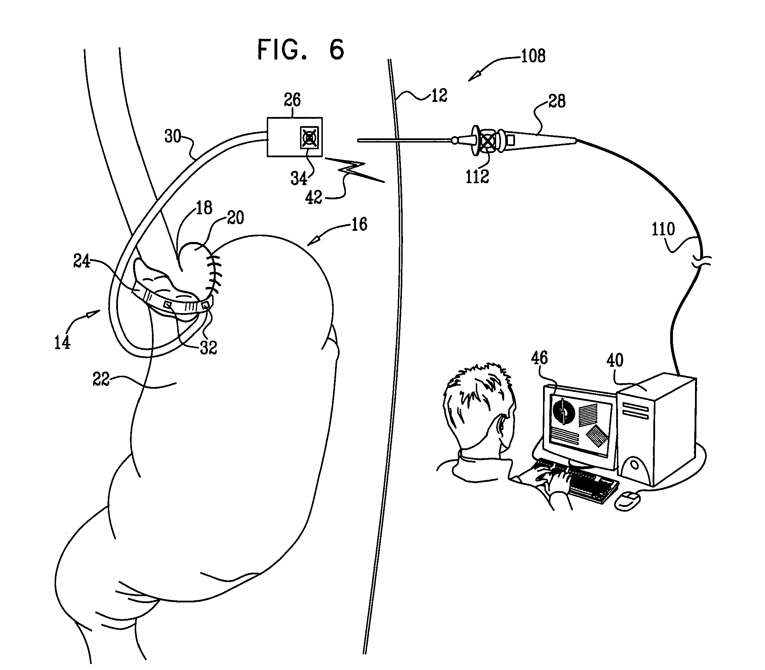

Reference is now made to Fig. 6, which schematically illustrates a system 108 for sensing a position and orientation of an injection or aspiration device relative to a port located in a living subject in accordance with an alternate embodiment of the invention. In this embodiment, the processor 40 is retrofitted to an existing tracking system, such as the Carto-Biosense^{®} Navigation System, available from Biosense Webster Inc., 3333 Diamond Canyon Road, Diamond Bar CA 91765. The processor 40 is designed to receive and process signals received over a cable 110 from one or more sensor coils in a transponder 112, using the signal processing circuitry 70 (Fig. 3) to determine the position and orientation of the transponder. The wire 110 may also conduct power signals to the transponder 112, which is constructed similar to the transponder 48 (Fig. 2), except that the power coil 50 can be omitted. The transponder 34 can be wireless, as shown in Fig. 6. Alternatively, it is also possible, but less convenient, for wires (not shown) leading from the transponder 34 to be brought out to the body surface 12 and connected to the processor 40, in which case a copy of the transponder 112 can be substituted for the transponder 34. In any case, the receiver 68 demodulates the signals generated by either or both of the transducers 34, 36 so as to reconstruct the variable current signals generated by respective instances of the sensing coil 52. The existing processing circuits use this information to determine the position and orientation of the transponders, just as if the sensor coil currents had been received by a wireless connection.

### Embodiment 6

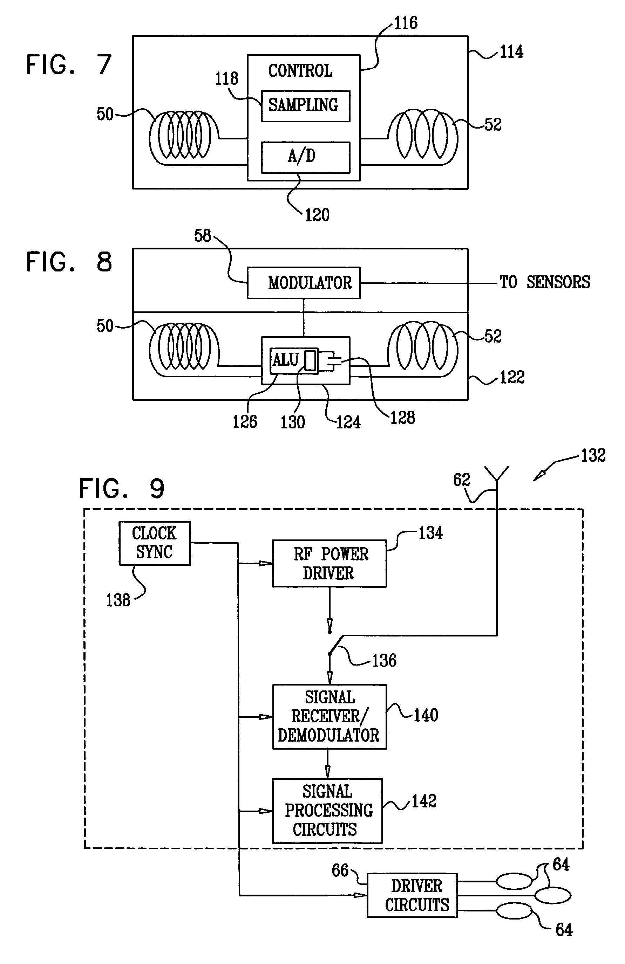

Reference is now made to Fig. 7, which schematically illustrates details of a wireless position transponder 114, which can be used as the transponders 34, 36 (Fig. 1), in accordance with an alternate embodiment of the invention. The transponder 114 is similar to the transponder 48 (Fig. 2), except that a control chip 116 includes a sampling circuit 118 and an analog-to-digital converter 120 (A/D), which digitizes the amplitude of the current flowing in the sensing coil 52. In this case, the control chip 116 generates a digitally modulated signal, and RF-modulates the signal for transmission by the power coil 50. Any suitable method of digital encoding and modulation may be used for this purpose. Other methods of signal processing and modulation will be apparent to those skilled in the art.

### Embodiment 7

Reference is now made to Fig. 8, which schematically shows details of a wireless transponder 122 in accordance with an alternate embodiment of the invention. The transponder 122 is similar to the transponder 48 (Fig. 2), except that a control chip 124 comprises an arithmetical logic unit 126 (ALU) and a power storage device, such as a capacitor 128, typically having a capacitance of about 1 microfarad. Alternatively, the power storage device comprises a battery or other power storage means known in the art. The entire transponder 122 is typically 2-5 mm in length and 2-3 mm in outer diameter.

The control chip 124 measures the voltage drop across the sensing coil 52 at different field frequencies, as explained hereinabove. Employing the arithmetical logic unit 126, the control chip 124 digitally encodes the phase and amplitude values of the voltage drop. For some applications, the measured phase and amplitude for each frequency are encoded into a 32-bit value, e.g., with 16 bits representing phase and 16 bits representing amplitude. Inclusion of phase information in the digital signal allows the resolution of the above-noted ambiguity that would otherwise occur in the signals when a 180 degree reversal of the sensing coil axis occurs. The encoded digital values of phase and amplitude are typically stored in a memory 130 in the control chip 124 using power supplied by the capacitor 128. The stored digital values are subsequently transmitted by the transponder 122 using a digital RF signal, as described hereinbelow. For some applications, the control chip 124 digitally encodes and transmits only amplitude values of the voltage drop across the sensing coil 52, and not phase values.

Reference is now made to Fig. 9, which schematically show details of the driving and processing circuitry 132, which are cooperative with the transponder 122 (Fig. 8), in accordance with a disclosed embodiment of the invention. The circuitry 132 comprises a RF power driver 134, which drives the antenna 62 to emit a power signal, typically in the megahertz range, e.g., about 13 MHz. An optional switch 136, embodied in hardware or software, couples the RF power driver 134 to the antenna 62 for the duration of the emission of the power signal. The power signal causes a current to flow in the power coil 50 of the transponder 122, which current is rectified by the control chip 124 and used to charge the capacitor 128. Typically, but not necessarily, the circuitry 132 includes a clock synchronization circuit 138, which is used to synchronize the RF power driver 134 and the driver circuitry 66. As mentioned hereinabove, the driver circuitry 66 drive the field generator coils 64 to generate electromagnetic fields. The electromagnetic fields cause a time-varying voltage drop across the sensing coil 52 of the transponder 122 (Fig. 8).

The digitally modulated RF signals transmitted by the transponder 122 (Fig. 8) is picked up by a receiver 140, which is coupled to the antenna 62 via the switch 136. The switch 136, shown connecting the receiver 140 to the antenna 62, can be decoupled from the receiver 140 to connect the antenna 62 with the RF power driver 134. The receiver 140 demodulates the signal to generate a suitable input to signal processing circuitry 142. The digital signals are received and used by the signal processing circuitry 142 to compute the position and orientation the transponder 122 (Fig. 8) as described above.

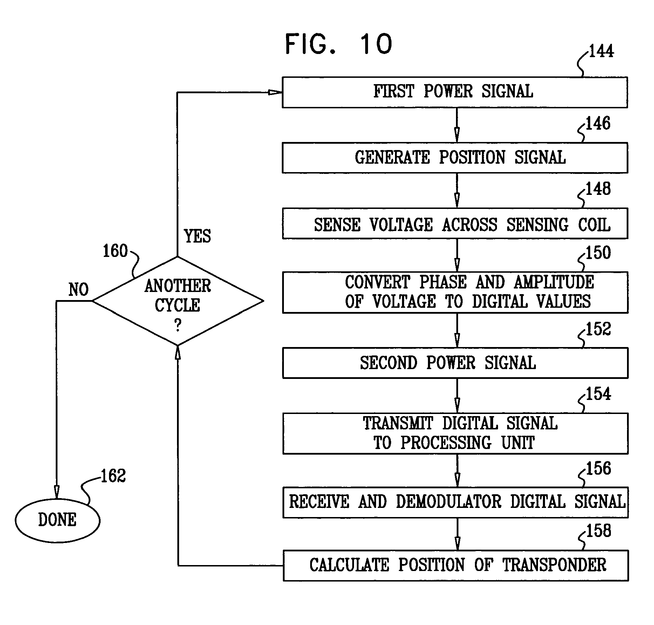

Reference is now made to Fig. 10, which is a flow chart that schematically illustrates a method for transmitting a digital signal, using the transponder 122 (Fig. 8) and the circuitry 132 (Fig. 9), in accordance with a disclosed embodiment of the invention. It is emphasized that the particular sequence shown in Fig. 10 is by way of illustration and not limitation, and the scope of the present invention includes other protocols that would be obvious to a person of ordinary skill in the art.

The method begins at initial step 144 in which the power driver 60 (Fig. 3) generates a first RF power signal, typically for about 5 milliseconds, which causes a current to flow in the power coil 50, thereby charging the capacitor 128 (Fig. 8). Subsequently, in step 146, the driver circuitry 66 drives the field generator coils 64 (Fig. 9) to produce electromagnetic fields, typically for about 20 milliseconds and thereby generate position signals.

At step 148 the fields generated in step 146 induce a voltage drop across the sensing coil 52 of the transponder 122, which is measured by the control chip 124.

Next, at step 150, using the power stored in the capacitor 128 (Fig. 8), the arithmetical logic unit 126 converts the amplitude and phase of the sensed voltage into digital values, and stores these values in the memory 130.

If the capacitor 128 is constructed such that at this stage it has largely been discharged, then at step 152, the power driver 60 generates a second RF power signal, typically for about 5 milliseconds, to recharge the capacitor 128. In applications in which the capacitor 128 retains sufficient charge to power the operations described below, step 152 can be omitted.

Next, at step 154 Using the stored energy, the control chip 124 generates a digitally modulated signal based on the stored digital values, and RF-modulates the signal for transmission by the power coil 50. Alternatively, the signal is transmitted using the sensing coil 52, for example, if a lower frequency is used. This transmission typically requires no more than about 3 milliseconds. Any suitable method of digital encoding and modulation may be used for this purpose, and will be apparent to those skilled in the art.

Next, at step 156, the receiver 140 receives and demodulates the digitally modulated signal.

Next, at step 158, the signal processing circuitry 142 uses the demodulated signal to compute the position and orientation of the transponder 122.

Control now proceeds to decision step 160, where it is determined whether another operation cycle the transponder 122 is to be performed. If the determination at decision step 160 is affirmative, then control returns to initial step 144. Typically, step 144 through step 158 are repeated continuously during use of the transponder 122 to allow position and orientation coordinates to be determined in real time.

If the determination at decision step 160 is negative, then control proceeds to final step 162, and the procedure terminates.

The process steps are shown in a linear sequence in Fig. 10 for clarity of presentation. Typically, the RF driving field is received and electrical energy stored in the transponder during a first time period, and the digital output signal is transmitted by the transponder during a second time period. However, it will be evident that these steps could be performed concurrently or in many different orders. In embodiments in which the method of Fig. 10 is performed using a plurality of transponders concurrently, the process steps may be interleaved among the different transponders in many different combinations.

### Embodiment 8

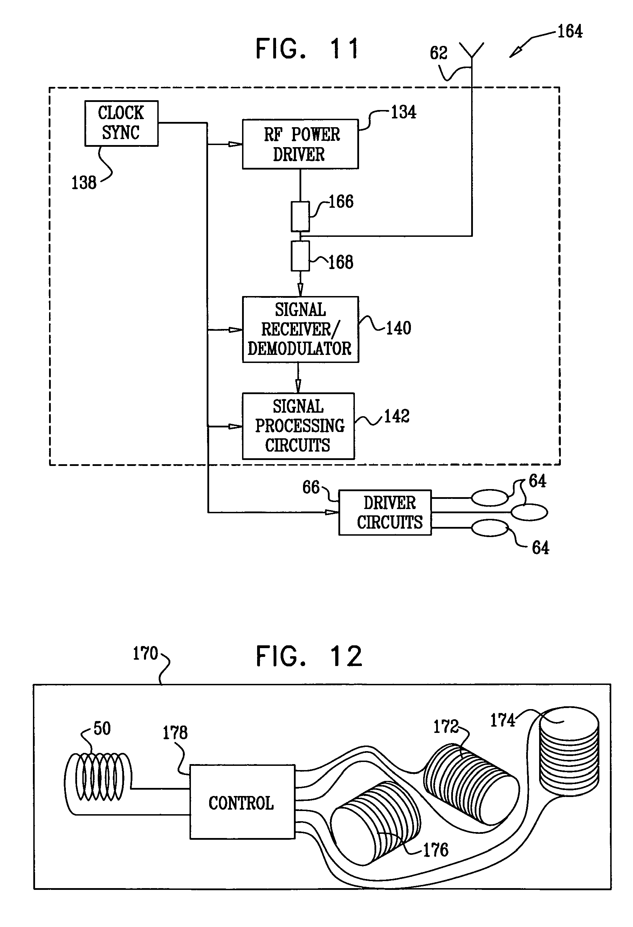

Reference is now made to Fig. 11, which schematically show details of driving and processing circuitry 164, which are cooperative with the transponder 122 (Fig. 8), in accordance with an alternate embodiment of the invention.

The circuitry 164 is similar to the circuitry 132 (Fig. 9), except that the switch 136 has been replaced by two band pass filters 166, 168. The band pass filter 166 couples the RF power driver 134 to the antenna 62, and, for example, may allow energy in a narrow band surrounding 13 MHz to pass to the antenna. The band pass filter 168 couples the receiver 140 to the antenna 62, and, for example, may allow energy in a narrow band surrounding 433 MHz to pass from the antenna to the receiver. Thus, RF power generated by the RF power driver 134 is passed essentially in its entirety to the antenna 62, and substantially does not enter circuitry of the receiver 140.

Further details of the embodiments shown in Figs. 8, 9, and 11 are disclosed in the above-noted U.S. Patent Application Publication No. 2005/0099290.

### Embodiment 9

Referring again to Fig. 3, in some applications, quantitative measurement of the position and orientation of the transponder to a reference frame is necessary. This requires at least two non-overlapping field generator coils 64 that generate at least two distinguishable AC magnetic fields, the respective positions and orientations of the field generator coils 64 relative to the reference frame being known. The number of radiators times the number of sensing coils is equal to or greater than the number of degrees of freedom of the desired quantitative measurement of the position and orientation of the sensors relative to the reference frame.

In the embodiment of Fig. 2, the single sensing coil 52 is generally sufficient, in conjunction with field generator coils 64, to enable the signal processing circuitry 70 to generate three dimensions of position and two dimensions of orientation information. The third dimension of orientation (typically rotation about the longitudinal axis) can be inferred if needed from mechanical information or, from a comparison of the respective coordinates of two transponders. However, in some applications, a larger number of degrees of freedom of the quantitative measurements is required.

Reference is now made to Fig. 12, which schematically illustrates details of a wireless position transponder 170, which can be used as the transponders 34, 36 (Fig. 1), in accordance with an alternate embodiment of the invention. The transponder 170 has a plurality of sensing coils 172, 174, 176, which are preferably mutually orthogonal, and are connected to a control chip 178. One of the axes of the sensing coils 172, 174, 176 may be conveniently aligned with the long axis of the device with which the transponder 170 is associated. The transponder 170 operates similarly to the transponder 48 (Fig. 2). However, the signal processing circuitry 70 (Fig. 3) can now determine all six position and orientation coordinates of the transponder 170 unambiguously.

The sensing coils 172, 174, 176 (and the sensing coil 52 (Fig. 2)) are preferably wound on air cores. The sensing coils 172, 174, 176 are closely spaced to reduce the size of the transponder 170, so that the transponder 170 is suitable for incorporation in a small device. The sensing coils can have an inner diameter of 0.5 mm and have 800 turns of 16 micrometer diameter to give an overall coil diameter of 1-1.2 mm. The effective capture area of each coil is preferably about 400 mm². It will be understood that these dimensions may vary over a considerable range and are only representative of a preferred range of dimensions. In particular, the size of the coils could be as small as 0.3 mm (with some loss of sensitivity) and as large as 2 or more mm. The wire size can range from 10-31 micrometers and the number of turns between 300 and 2600, depending on the maximum allowable size and the wire diameter. The effective capture area should be made as large as feasible, consistent with the overall size requirements. While the preferred sensor coil shape is cylindrical, other shapes can also be used. For example a barrel shaped coil can have more turns than a cylindrical shaped coil for the same diameter of implant. Also, square or other shaped coils may be useful depending on the geometry of the catheter.

A plurality of sensing coils may optionally be incorporated, *mutatis mutandis,* in the transponder 114 (Fig. 7) and the transponder 122 (Fig. 8).

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. A method for adjusting an inflatable gastric restriction device within a body of a living subject, comprising the steps of:
disposing a wireless transponder on a gastric restriction device, said wireless transponder generating a location signal relative to a receiver, said receiver having a known relation to an injection device that is adapted to a port of said gastric restriction device;
irradiating said wireless transponder with a driving field; said wireless transponder being powered at least in part by said driving field;
responsively to said driving field wirelessly transmitting an output signal by said wireless transponder;
receiving and processing said output signal to determine respective locations and orientations of said injection device and said port;
responsively to said respective locations and orientations navigating said injection device within said body to introduce said injection device into said port; and
changing a fluid content of said gastric restriction device using said injection device.

2. The method according to claim 1, further comprising the steps of:
disposing a second wireless transponder that generates a second output signal on said injection device; and
generating a plurality of electromagnetic fields at respective frequencies in a vicinity of said wireless transponder and in a vicinity of said second wireless transponder, wherein said output signal and said second output signal include information indicative of respective strengths of said electromagnetic fields at said wireless transponder and said second wireless transponder.

3. The method according to claim 2, wherein one of said output signal and said second output signal is a digital output signal.

4. The method according to claim 2, further comprising the steps of:
storing first electrical energy and second electrical energy derived from said driving field in said wireless transponder and said second wireless transponder, respectively; and
transmitting said output signal and said second output signal using said first electrical energy and said second electrical energy, respectively.

5. The method according to claim 2, wherein said wireless transponder and said second wireless transponder are powered exclusively by said driving field.

6. The method according to claim 1, wherein said driving field is a radiofrequency driving field.

7. The method according to claim 1, wherein said driving field is an acoustic energy field.

8. The method according to claim 1, wherein said output signal is frequency modulated.

9. The method according to claim 1, further comprising the step of transmitting telemetry signals from said gastric restriction device, said telemetry signals containing information of a state of said gastric restriction device.

10. A location system for adjusting an inflatable gastric restriction device within a body of a living subject, said gastric restriction device having a port and a first wireless transponder, the system comprising:
an injection device that is receivable by said port of said gastric restriction device, said injection device having a second wireless transponder, said first wireless transponder and said second wireless transponder each comprising a position sensor;
a transmitter for irradiating said first wireless transponder and said second wireless transponder with a driving field; said first wireless transponder and said second wireless transponder each being powered at least in part by said driving field to energize said position sensor thereof;
wherein said first wireless transponder and said second wireless transponder are operative responsively to said driving field for wirelessly transmitting a first output signal and a second output signal, respectively;
electrical circuitry for receiving and processing said first output signal and said second output signal to determine respective locations and orientations of said port and said injection device; and
a console that is operative for displaying visual indications of said respective locations and orientations.

11. The location system according to claim 10, wherein said transmitter is operative to generate a plurality of electromagnetic fields at respective frequencies in a vicinity of said first wireless transponder and in a vicinity of said second wireless transponder, wherein said first output signal and said second output signal include information indicative of respective strengths of said electromagnetic fields at said first wireless transponder and said second wireless transponder.

12. The location system according to claim 10, wherein said first wireless transponder and said second wireless transponder are powered exclusively by said driving field.

13. The location system according to claim 10, wherein said driving field is a radiofrequency driving field.

14. The location system according to claim 10, wherein said driving field is an acoustic energy field.

15. The location system according to claim 10, further comprising:
a plurality of field generators, adapted to generate electromagnetic fields at respective frequencies in a vicinity of said gastric restriction device and said port;
wherein said first wireless transponder and said second wireless transponder each comprises:
a power coil, coupled to receive said driving field;
a power storage device, adapted to store electrical energy derived from said driving field;
at least one sensor coil, coupled so that a voltage drop is induced across said sensor coil responsive to the electromagnetic fields; and
a control circuit, coupled to said sensor coil and to said power storage device, and adapted to use said stored electrical energy, wherein said first output signal and said second output signal are respectively indicative of said voltage drop.

16. The location system according to claim 15, wherein said sensor coil comprises a plurality of mutually orthogonal sensor coils.

17. The location system according to claim 15, wherein said control circuit comprises a voltage-to-frequency converter, wherein a frequency of a control circuit output signal thereof is proportional to said voltage drop.

18. The location system according to claim 15, wherein said control circuit comprises an arithmetical logic unit adapted to digitally encode an amplitude of said voltage drop in a control circuit output signal thereof.

19. The location system according to claim 18, wherein said arithmetical logic unit is further adapted to digitally encode a phase of said voltage drop in said control circuit output signal.

20. The location system according to claim 15, wherein said control circuit comprises a sampling circuit and an analog-to-digital converter operative for digitizing an amplitude of a current flowing in said sensor coil, and for digitally modulating a control circuit output signal thereof.

21. The location system according to claim 10, further comprising: a telemetry receiving unit for receiving telemetry signals transmitted from said first wireless transponder, said telemetry signals containing information of a state of said gastric restriction device.
